# EUROPEAN PATENT SPECIFICATION

(11) **EP 1 996 985 B1**
(45) Date of publication and mention of the grant of the patent: **23.12.2009**
(21) Application number: 07711810.7
(22) Date of filing: 07.03.2007
(51) Int. Cl.: G05B 21/00, G06F 19/00, G01N 33/483, G01N 33/50

(54) **SAFETY APPROACH FOR DIAGNOSTIC SYSTEMS**
SICHERHEITSMETHODE FÜR DIAGNOSTISCHE SYSTEME
APPROCHE SÉCURITÉ DE SYSTÈMES DE DIAGNOSTIC

(30) Priority: 09.03.2006 EP 06004807
(43) Date of publication of application: 03.12.2008
(73) Proprietor: Roche Diagnostics GmbH, 68305 Mannheim (DE); F.HOFFMANN-LA ROCHE AG, 4070 Basel (CH)
(72) Inventor: FRIEDRICHS, Juergen, 82380 Peissenberg (DE); PINSL-OBER, Judith, 82327 Tutzing (DE)
(86) International application number: PCT/EP2007/001936
(87) International publication number: WO 2007/101662

(56) References cited:
- WO-A-2004/011904
- US-A- 5 591 573
- US-B1- 6 649 339
- VENKATASUBRAMANIAN V: "Prognostic and diagnostic monitoring of complex systems for product lifecycle management: Challenges and opportunities" COMPUTERS & CHEMICAL ENGINEERING, PERGAMON PRESS, OXFORD,, GB, vol. 29, no. 6, 15 May 2005 (2005-05-15), pages 1253-1263, XP004885026 ISSN: 0098-1354

## Description

### Field of invention

The present invention provides a diagnostic safety method, a diagnostic device, a computer program and a diagnostic system for analyzing a plurality of samples with regard to the presence of contaminations.

### Background of the Invention

Contamination during analytic procedures and of products is a common problem in many fields of science, health care and industry. Especially for the food industry and for medical services, the quality control regulations demand that the existence of germs like bacteria, virus and fungi has to be controlled strictly, even though an environment completely free of germs is not achievable. For IVD devices contaminations need to be avoided to circumvent infections of patients as well as to guarantee a proper diagnosis. With respect to science such biological contaminations may have drastic effects on measurements leading to false results.

Due to the increasing demand for enhancing the amount of products and processes, a high degree of automation and parallelization is required in industry and health care that devotes high demands on the precision and reliability of instrumentation and automation, especially based on the operational desire to optimize sample volumes and the time-to-result, while at the same time to provide high sensitivity and low failure rates.

Current diagnostic systems for IVD and medical application utilize specific safety measures to detect "single failures", which could potentially result in malfunction und misdiagnosis. This specific safety approach is based on identifying the most critical processing steps bearing the highest potential to create false results in case of a malfunction or failure, and to enhance the reliability of this process step by adding specific test equipment for failure detection. Examples are e.g. self tests for individual sensors (light barriers, temperature sensors, motors, controllers), the surveillance of pipetting steps (aspiration and dispensing volume) or the process temperatures. Consequently, these approaches have the drawback that only certain parts of the system are assured by adding accessory equipment.

A control system for a dialysis machine is described in US 6,868,309. WO 01/14593 describes a method to produce quality assured biological samples and US 5,591,573 describes a method to test blood samples. Mortimer, J., describes pooling strategies to test donated blood for viral genomes (Vox Sang 73 (1997) 93-96).

For other safety critical applications in the area of e.g. railways or air traffic a redundant, multi channel architectures is applied (Graband, M., et al, Signal+Draht 80 (1988) 199-204), whereas the comparison of the redundant channel results is used to detect one or more failures. Here, the entire system is assured by the safety approach.

The present invention provides a new safety approach for diagnostic systems that satisfies the demands for safety of the diagnostic result as well as throughput.

### Summary of the Invention

The present invention is directed to a safety method for a diagnostic system, a diagnostic device, a diagnostic system and a computer program capable of analyzing samples.

One aspect of the present invention is a safety method for a diagnostic system that analyzes a plurality of samples with regard to the presence of contaminations comprising the steps
a) taking an aliquot of a first sample,
b) dividing said aliquot into at least two aliquot parts,
c) analyzing each of said at least two aliquot parts with respect to the presence of contaminations simultaneously and
d) comparing the sample results obtained for each of said at least two aliquot parts in step c) in order to verify the presence or absence of contaminations in said sample and/or to detect failures of the diagnostic system, wherein
   steps a) to d) are repeated with another sample from said plurality of samples until all samples of said plurality of samples are analyzed.

Throughout the present invention a diagnostic system provides a diagnostic result and it is of course of utmost importance that this diagnostic result is reliable. Therefore, special precautions have to be taken to maximize the result safety of said diagnostic system, while maintaining a suitable throughput, cost and sample volume. The safety method of the present invention can e.g. provide sample results with high safety without affecting neither the throughput nor the required sample volume. In the present invention the phrase "sample result" summarizes all kinds of measurement results that can be obtained with diagnostic techniques known to someone skilled in the art. Examples for sample results are numeric results like a mass of an analyte obtained by mass spectrometry or a set of measurement values like a growth curves obtained by real-time PCR.

The safety method of the present invention analyzes samples with regard to the presence of contaminations. Depending on the application of the safety method, such contaminations are biological contaminations such as bacteria, viruses, fungi, proteins or nucleic acids or chemical contaminations such as toxic agents. At the end, the safety method has to verify, if the analyzed sample comprises a certain contamination or not.

In addition to the verification of the presence or absence of contaminations in a certain sample, the safety method of the present invention also detects failures of the diagnostic system. In general, a failure of the diagnostic system is detected, if the comparison of certain results does not fulfill defined criteria. In such a case, th sample is not named "contaminated" (reactive) or "non contaminated" (nonreactive), but, depending on the application, the analysis can e.g. be repeated, disregarded or tested with other methods. In certain embodiments of the present invention, the failures of the diagnostic system are recorded in order to detect recurring errors.

Although the safety method of the present invention can also be performed with a single sample, its power is exploited, if a plurality of samples is to be tested, because the throughput can be maintained, while a multiplicate analysis improves the safety of the method.

The safety method of the present invention is based on the principal that the aliquot of a certain sample is divided into at least two aliquot parts. These at least two identical aliquot parts are than analyzed simultaneously in order to provide redundant diagnostic results. For the performance of the present invention it is not only necessary that the at least two simultaneous analysis are performed at the same point of time, but also with the identical diagnostic device hardware.

Therefore, for each of said aliquot parts a separate diagnostic device or a separate processing lane within a single diagnostic device has to be provided. Only if the comparability of the at least two sample results is assured, it is possible to improve the safety of the analysis. Note that the partition of an initial aliquot into at least two aliquot parts has an impact on the sensitivity of the method, because every aliquot part comprises only a respective part of a potential contamination. If this loss in sensitivity is of importance for a certain application, the effect can be at least partially compensated by special combination procedures that are explained later on.

The safety method is repeated with another sample until all samples of said plurality of samples are analyzed.

Another aspect of the present invention is a safety method for a diagnostic system that analyzes a plurality of samples with regard to the presence of contaminations comprising the steps
a) providing a blent sample of aliquots taken from each member of a group of samples chosen from said plurality of samples,
b) dividing said blent sample into at least two aliquot parts,
c) analyzing each of said at least two aliquot parts with respect to the presence of contaminations simultaneously and
d) comparing the sample results obtained for each of said at least two aliquot parts in step c) in order to verify the presence or absence of contaminations in said group of samples and/or to detect failures of the diagnostic system, wherein
   steps a) to d) are repeated with another group of samples from said plurality of samples until all samples of said plurality of samples are analyzed.

In this embodiment of the safety method according to the present invention, blent samples are analyzed. Such a blent sample comprises aliquots from a certain number of samples, whereas from each sample an aliquot with preferably the same volume is used. Each sample that contributes with an aliquot to a certain blent sample is a member of the respective group of samples and each sample of said plurality of samples is a member of only one group of samples. Note that the use of blent samples increases the sample throughput, but reduces the information about contaminations from a single sample to a group of samples. This embodiment of the present information is therefore useful for applications, where large numbers of samples need to be analyzed. If a contamination is detected in a group of samples, the entire group is marked as contaminated. Optionally, each of the group members can subsequently be analyzed separately in order to find the one or more contaminated sample of said group.

Yet another aspect of the present invention is a diagnostic device capable of analyzing samples with regard to the presence of contaminations comprising
A) at least two identical processing lanes each equipped with a sample input means and means for the analysis of said samples in order to obtain redundant diagnostic results of a sample and
B) means for the comparison of said redundant diagnostic results of the at least two identical processing lanes in order to verify the presence or absence of contaminations in said sample and/or to detect failures of the diagnostic device.

A diagnostic device capable of analyzing samples with regard to the presence of contaminations according to the present invention has at least two identical processing lanes to produce redundant diagnostic results of a sample. Only if the processing lanes of said diagnostic device are identical, the at least two redundant sample results can be used to improve the safety of the analysis.

Still another aspect of the present invention is a computer program executable by a diagnostic device to analyze samples with regard to the presence of contaminations comprising the steps
aa) obtaining at least two redundant diagnostic results of a sample with respect to the presence of contaminations,
bb) comparing said redundant diagnostic results in order to verify the presence or absence of contaminations in said sample and/or to detect failures of the diagnostic device.

A further aspect of the present invention is a diagnostic system to analyze samples with regard to the presence of contaminations comprising
AA) a diagnostic device and
BB) a computer program according to the present invention.

### Description of the Figures

Figure 1: Flowchart picture of the safety method according to the present invention.
Figure 2: Schematic plots illustrating the sensitivity regain.

### Detailed description of the invention

One aspect of the present invention is a safety method for a diagnostic system that analyzes a plurality of samples with regard to the presence of contaminations comprising the steps
a) taking an aliquot of a first sample,
b) dividing said aliquot into at least two aliquot parts,
c) analyzing each of said at least two aliquot parts with respect to the presence of contaminations simultaneously and
d) comparing the sample results obtained for each of said at least two aliquot parts in step c) in order to verify the presence or absence of contaminations in said sample and/or to detect failures of the diagnostic system, wherein
steps a) to d) are repeated with another sample from said plurality of samples until all samples of said plurality of samples are analyzed.

A preferred safety method according to the present invention is a method, wherein each sample comprises an internal control and said analyzing in step c) provides additional control results for each aliquot part that are optionally compared in step d) in addition to the comparison of said sample results in order to verify the presence or absence of contaminations in said sample and/or to detect failures of the diagnostic system.

Throughout the present invention an internal control is a compound that is present in the sample from the beginning or that is spiked to the sample prior to the analysis. Such an internal control provides additional control results during the sample analysis in step c). In both case it is of importance to know the exact amount of said compound in the sample or at least to know the expected control result. If the obtained control result is within a certain range of the expected control result, the control result is called "valid" throughout the present invention.

In another preferred safety method according to the present invention, said analyzing of each of said at least two aliquot parts in step c) is performed simultaneously on identical analyzers or simultaneously on one analyzer having at least two identical processing lanes.

As mentioned before, it is necessary that the at least two simultaneous analyses are performed not only at the same point of time, but also with identical diagnostic device hardware. This can be assured by providing identical analyzers or one analyzer having at least two identical processing lanes. Identical processing lanes need to have identical sample input means and identical means for the analysis of said samples in order to obtain sample results. Results obtained in such a manner are called "redundant results" throughout the present invention.

In an also preferred safety method according to the present invention, said additional control results are obtained simultaneously on each identical analyzer or on each of said at least two identical processing lanes.

Due to the use of internal controls, the additional control results are obtained simultaneously during the analysis of the each aliquot part on identical analyzers or on identical processing lanes of one analyzer. In other words, for each sample result an additional control result is obtained.

In yet another preferred safety method according to the present invention, said aliquot of step a) is divided into 2 - 4 aliquot parts and said analyzing in step c) is performed simultaneously on 2 - 4 identical analyzers or simultaneously on one analyzer having 2 - 4 identical processing lanes.

Due to the sensitivity issue mentioned before, it does not make sense to divide the initial aliquot of a sample into a large number of aliquot parts, because the contamination will soon reach an undetectable amount. Moreover, for each aliquot part an identical analyzer or an identical processing lane of one analyzer must be provided. Therefore, it is preferred to divide the initial aliquot into 2 - 4 aliquot parts.

Another preferred safety method according to the present invention is a method, wherein in step a) said aliquot is a blent sample of aliquots taken from each member of a group of samples chosen from said plurality of samples, said blent sample is divided into at least two aliquot parts in step b) and the comparison of sample results in step d) is performed in order to verify the presence or absence of contaminations in said group of samples and/or to detect failures of the diagnostic system.

As mentioned before, such a blent sample comprises aliquots from a certain number of samples, whereas the aliquot from each sample has preferably the same volume and each sample that contributes with an aliquot to a certain blent sample is a member of the respective group of samples, whereas each sample of said plurality of samples is a member of only one group of samples.

In a more preferred safety method according to the present invention, said group of samples comprises 2 - 4 samples from said plurality of samples and/or said blent sample is divided into 2 - 4 aliquot parts.

Said blent samples can be used in different ways. If a certain group of samples has 3 members, then a blent sample comprising e.g. equal aliquots from each member can be divided into 3 aliquot parts each comprising 1/3 of each initial aliquot. On the other hand a blent sample from a group of 3 members can also be divided into 2 aliquot parts each with 1/2 of each initial aliquot. To summarize, the number of members per group of samples as well as the number of aliquot parts can freely be chosen in order to obtain a desired throughput, sample resolution and sensitivity.

In another more preferred safety method according to the present invention, the steps a) to d) are repeated with another group of samples from said plurality of samples until all samples of said plurality of samples are analyzed.

Within the present invention it is not necessary that said another group of samples has the same number of members as the first group of samples or that the number of aliquot parts is the same for each group of samples.

Based on the sample results and optionally the control results, the safety method according to the present invention can decide between three different situations after the analysis of a sample or a group of samples, namely the presence of a contamination, the absence of a contamination or the failure of the analysis.

In a preferred safety method according to the present invention, the presence of contaminations is verified, if
(i) each analysis in step c) provides a sample result and said sample results compared in step d) are equal or
(ii) each analysis in step c) provides a sample result, said sample results compared in step d) are different and the control results are valid or
(iii) not all analyses in step c) provide a sample result and the control results are valid.

In another preferred safety method according to the present invention, a failure of the diagnostic system is detected, if
(i) the sample results from step c) that are compared in step d) are different or
(ii) not all control results are valid.

In yet another preferred safety method according to the present invention, the absence of contaminations is verified, if no analysis in step c) provides a sample result and the control results are valid.

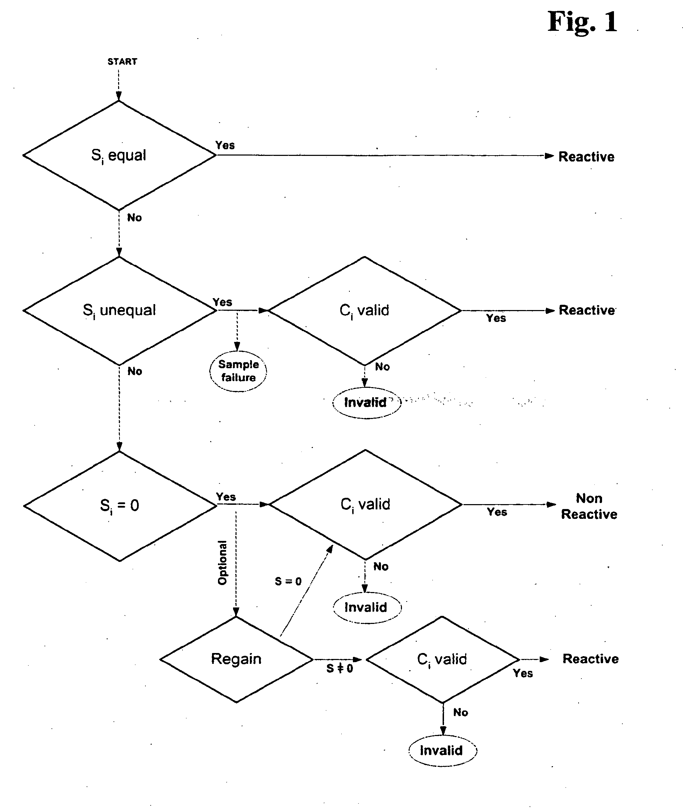

The consequences of the different combinations of sample results (Sᵢ) and control results (Cᵢ) are summarized in Figure 1. Note that the sample results are equal (Sᵢ equal) throughout the present invention, if the difference between each of the sample results is below a certain defined threshold. Consequently, if the difference between at least one pair of sample results is too large, the sample results are different (Sᵢ unequal) corresponding to a failure that is named "sample failure" throughout the present invention. Moreover, the sample results are unequal, if not all analyses in step c) provide a sample result and a certain number of analyses provide no sample result.

With respect to the control results there are two different situations. Because the expected control result is know, the control result is named "valid" (Cᵢ valid), if its difference to the expected control result is below a certain defined threshold. Consequently, if the difference from the expected control result is too large, the control result is "invalid" corresponding to a failure that is named a control failure throughout the present invention.

If no analysis in step c) provides a sample result (Sᵢ = 0) and the control results are valid, no contamination is detected and the sample or the group of samples is "non reactive".

Another preferred safety method according to the present invention is a method, wherein detected failures of the diagnostic system are recorded to produce failure histories for the diagnostic system and/or to calculate failure rates, wherein the entire diagnostic system is excluded, if failure rates exceed a defined threshold.

A more preferred safety method according to the present invention is a method, wherein control failures are recorded separately for each identical diagnostic device or for each identical processing lanes of one diagnostic device.

The recording of control failures and sample failures in failure histories can be used for different important statements with respect to the performance of the diagnostic device. If the detected control failures are recorded for the at least two diagnostic devices or the at least two processing lanes of one diagnostic device separately, it can be verified, if a high failure rate of the analysis procedure is mainly associated with one diagnostic devices or processing lane.

Using multiple failure histories, the detected failures can be correlated e.g. with reagent lots in order to figure out, if a high failure rate is associated with a certain reagent lot that can then be excluded from further analysis. Moreover, other embodiments of failure correlation are possible within the scope of the present invention depending on the detection method or the device setup. If the diagnostic device is e.g. based on a multiwell plate processing, it is possible to correlate the detected failures with the well position within the plates.

As mentioned before, the partition of an initial aliquot into at least two aliquot parts has an impact on the sensitivity of the method, because every aliquot part comprises only a respective part of a potential contamination. If this loss in sensitivity is of importance for a certain application, the effect can be at least partially compensated by combination of the obtained sample results.

Yet another preferred safety method according to the present invention further comprises a combination step e), wherein the sample results obtained for each analysis in step c) are combined to verify the presence or absence of contaminations in said sample.

The phrase "combination of sample results" is used throughout the present invention to express that the sample results obtained from each analysis in step c) are combined in order to make a single statement with respect to the presence of contaminations. For this combined approach, the amount of data points to detect a potential contamination is increased by the combination of the sample results obtained in step c), whereas each sample result is treated as an independent analysis result. As a consequence, the statistical weight of the analysis is increased and the standard deviation of the measurement is reduced (see e.g. Lorenz, "Grundbegriffe der Biometrie", Gustav Fischer Verlag, 1996).

In a more preferred safety method according to the present invention, said combination of sample results increases the sensitivity of the safety method.

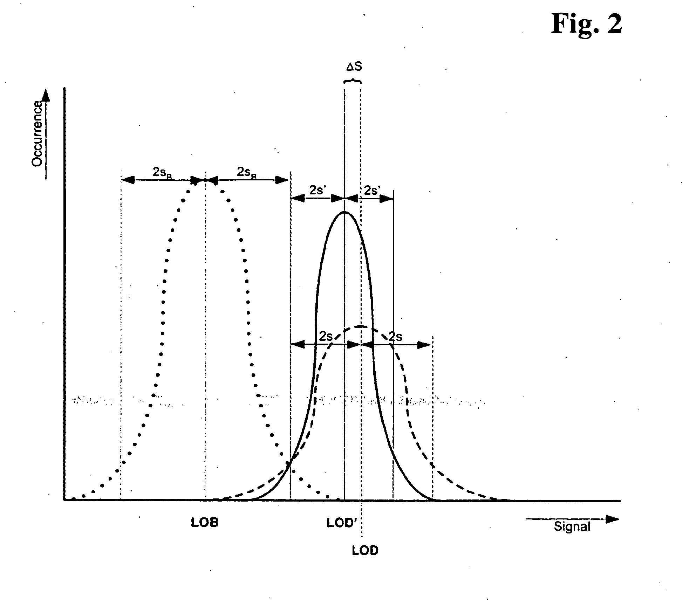

The increase in sensitivity (sensitivity gain ΔS) due to the combination of sample results and the corresponding reduction of the sample result distribution is schematically illustrated in Figure 2. In general, the sensitivity of any analysis procedure is limited due to the background of the analytic technique used ("limit of blank" (LOB)). The respective background signal has a distribution (dotted line in Figure 2) characterized by a standard deviation (2s_{B}).

Since the sample result has a distribution characterized by a standard deviation, too, the limit of detection (LOD) is defined as the signal threshold under which the respective 2s-intervals of the background distribution and the sample result distribution starts to overlap. Consequently, if the 2s-interval of the sample result can be reduced due to the increase of data points, the LOD can be shifted to lower values. This effect is illustrated by the solid and the dotted line in Figure 2. Here, the LOD (broken line) could be reduced to LOD' (solid line) due to the reduction of the distribution width from 2s (broken line) to 2s' (solid line) corresponding to a sensitivity gain of ΔS.

In another more preferred safety method according to the present invention, said combination of sample results comprises an additional hardware compensation step.

Throughout the present invention, the sample results that are combined for the optional sensitivity regain are obtained on different diagnostic devices or on different processing lanes of one diagnostic device. Although these diagnostic devices or processing lanes are supposed to be identical, there will be slight differences in their background signals. Therefore, the sample results can be corrected for these hardware differences in order to ensure the validity of result combination for sensitivity regain. This correction for hardware differences between different diagnostic devices or between different processing lanes is called hardware compensation step throughout the present invention.

The safety method according to the present invention can be used for all kinds of samples that may have contaminations. The purpose for the detection of contaminations in a plurality of samples is e.g. quality control issues, pollution analysis of resources or pandemic screening.

In a preferred safety method according to the present invention, said samples are food samples or environmental samples.

In another preferred safety method according to the present invention, said samples are blood samples.

In case of blood screening applications the detection of contaminations is twofold. On the one hand, blood donations must be free from contaminations for further processing and on the other hand, the donor should be informed and/or excluded from further blood donations.

In yet another preferred safety method according to the present invention, said contaminations are microbial contaminations, preferably viruses, fungi or bacteria.

Diagnostic analysis of samples is preferably performed with respect to biological issues and the contaminations are viruses, fungi or bacteria. If such microbial contaminations have to be detected, the safety method of the present invention I general comprises additional steps for sample preparation, namely e.g. the lysis of cells or an isolation step to separate proteins or nucleic acids from the cell debris.

In still another preferred safety method according to the present invention, said analyzing in step c) is a PCR amplification.

If microbial contaminations like viruses, fungi or bacteria have to be detected, it is preferred to detect them via their characteristic nucleic acids. Since these nucleic acids are usually present in very small concentrations, it is necessary to perform an amplification reaction, such as a PCR amplification.

In a more preferred safety method according to the present invention, said PCR amplification is performed in real-time using SYBR Green, Taqman probes or hybridization probes.

Using a real-time PCR amplification has the well known advantage that the amplification of nucleic acids present in the sample can be monitored in real-time resulting in a growth curve. In general, real-time PCR is performed using fluorescent labeled probes, whereas the corresponding growth curves comprise a fluorescence value for each amplification cycle and a nucleic acid is detected, if the fluorescence increases significantly during the amplification process. In an embodiment of the present invention using a real-time PCR amplification for the analysis of step c), the obtained sample result is the cycle number at which the fluorescence intensity rises significantly over the fluorescence background (c_{T}-value) or the slope of the growth curve in a certain area.

The sample results of this embodiment of the present invention are equal, if the c_{T}-values or the growth curve slope of each amplification reaction are equal within boarders. Using the growth curve slope in order to verify the presence or absence of a contamination in a sample, one has to define a certain minimum slope that is indicative for the presence of a contamination. The slope itself is obtained by linear regression of the growth curve data points and therefore, it is afflicted with a certain uncertainty. This uncertainty can be reduced, if the data points from several growth curves are combined and consequently, the minimum slope that is indicative for the presence of a contamination can be lowered.

Moreover, in another preferred safety method according to the present invention, said analyzing in step c) is performed using array or ELISA assays.

Note that the analytical techniques discussed above are not limiting the scope of the invention and all analytical techniques suitable for the verification of a contamination within a sample known to someone skilled in the art can be used for the safety method according to the present invention.

Another aspect of the present invention is a diagnostic device capable of analyzing samples with regard to the presence of contaminations comprising
A) at least two identical processing lanes each equipped with a sample input means and means for the analysis of said samples in order to obtain redundant diagnostic results and
B) means for the comparison of said redundant diagnostic results of the at least two identical processing lanes in order to verify the presence or absence of contaminations in said sample and/or to detect failures of the diagnostic device.

A diagnostic device according to the present invention comprises at least two identical processing lanes. As mentioned before, although these processing lanes are supposed to be identical, there will be slight differences that will alter their background signals that can be corrected by a hardware compensation step.

Nevertheless, in order to produce redundant diagnostic results for said verification of presence or absence of contaminations, it is necessary that said at least two identical processing lanes are as independent as possible and therefore, each processing lane is equipped with at least its own sample input means and means for the analysis of samples. In addition, it is possible to equip each processing lane with its own power supply.

In a preferred diagnostic device according to the present invention, said at least two identical processing lanes are independent in order to produce redundant diagnostic results.

The diagnostic device according to the present invention has one means to compare the redundant diagnostic results from each identical processing lane. It is preferred that this means for comparison is a computer processor and that the diagnostic device has an additional means for data presentation, preferably a monitor.

In a preferred diagnostic device according to the present invention, said means for the comparison of redundant diagnostic results is a computer processor.

A preferred diagnostic device according to the present invention further comprises means for data presentation.

The samples that can be analyzed with the diagnostic device according to the present invention and the contaminations that can be detected were already discussed before.

In another preferred diagnostic device according to the present invention, said samples are food samples or environmental samples.

In yet another preferred diagnostic device according to the present invention, said samples are blood samples.

In still another preferred diagnostic device according to the present invention, said contaminations are microbial contaminations, preferably viruses, fungi or bacteria.

Within the scope of the present invention all analytical techniques suitable for the verification of a contamination within a sample known to someone skilled in the art can be used as means for the analysis of samples for the diagnostic device according to the present invention.

In a further preferred diagnostic device according to the present invention, said at least two identical processing lanes are able to perform real-time PCR amplifications.

In a preferred diagnostic device according to the present invention, said at least two identical processing lanes are able to perform array or ELISA assays.

Yet another aspect of the present invention is a computer program executable by a diagnostic device to analyze samples with regard to the presence of contaminations comprising the steps
aa) obtaining at least two redundant diagnostic results of a sample with respect to the presence of contaminations,
bb) comparing said redundant diagnostic results in order to verify the presence or absence of contaminations in said sample and/or to detect failures of the diagnostic device.

The computer program of the present invention is configured such that a diagnostic device performs said steps aa) and bb) in order to analyze samples with regard to the presence of contamination.

In a preferred embodiment of the computer program, said diagnostic device is a diagnostic device according to the present invention.

As mentioned before, a diagnostic device according to the present invention has at least two identical processing lanes each equipped with a sample input means and means for the analysis of said samples in order to obtain redundant diagnostic results.

In still another preferred embodiment of the computer program according to the present invention, additionally at least 2 redundant control results are provided.

Beside the redundant diagnostic results the computer program according to the present invention further requires at least 2, preferably 2 - 4 redundant control results to verify the presence or absence of contaminations in said sample and to detect failures of the diagnostic device.

In another embodiment of the computer program according to the present invention, preferably 2 - 4 redundant diagnostic results are compared in step bb).

Each redundant diagnostic result for said computer program implies that the diagnostic device has to become more complex in order to provide an additional redundant diagnostic result. Therefore, it is preferred to use a computer program that obtains 2 - 4 redundant diagnostic results.

In another preferred embodiment of the computer program according to the present invention, said redundant diagnostic results of a sample are the results of real-time PCR amplifications.

In yet another preferred embodiment of the computer program according to the present invention, said redundant diagnostic results of a sample are the results of array or ELISA assays.

Within the scope of the present invention all analytical techniques suitable for the verification of a contamination within a sample known to someone skilled in the art can be used to obtain the redundant diagnostic results with a computer program according to the present invention.

In a preferred computer program according to the present invention, the presence of contaminations is verified, if
(i) said redundant diagnostic results compared in step bb) are equal or
(ii) said redundant diagnostic results are different and said redundant control results are valid or
(iii) not all redundant diagnostic results are obtained and said redundant control results are valid.

In another preferred computer program according to the present invention, a failure of the diagnostic device is detected, if
(i) said redundant diagnostic results compared in step bb) are different or
(ii) not all redundant control results are valid.

In yet another preferred computer program according to the present invention, the absence of contaminations is verified, if no redundant diagnostic result is obtained and said redundant control results are valid.

The consequences of the different combinations of redundant diagnostic results and redundant control results were discussed already with respect to the safety method according to the present invention and apply here correspondingly. In brief, note that the redundant diagnostic results are equal, if the difference between each of the redundant diagnostic results is below a certain defined threshold. If the difference between at least one pair of diagnostic results is too large, the redundant diagnostic results are different corresponding to a failure that is named a "sample failure" throughout the present invention. With respect to the redundant control results there are two different situations. Because the expected control result is know, the redundant control result is named "valid", if its difference to the expected control result is below a certain defined threshold. If the difference from the expected control result is too large, the redundant control result is "invalid" corresponding to a failure that is named a control failure throughout the present invention.

A preferred computer program according to the present invention is a computer program, wherein detected failures of the diagnostic device are recorded to produce a failure history of the diagnostic device and/or to calculate a failure rate, wherein the entire diagnostic device is excluded, if said failure rate exceeds a defined threshold.

The advantages of recording the detected failures to produce a failure history of the diagnostic device were outlined before. In brief, the recording of multiple failure histories gives the opportunity to correlate the failures to certain aspects of the diagnostic device or to steps of the detection procedure in order to exclude said aspects or steps from further analysis.

Another preferred computer program according to the present invention further comprises a combination step cc), wherein redundant diagnostic results obtained in step aa) are combined to verify the presence or absence of contaminations in said sample.

As mentioned before, such a combination step can be used to increase the sensitivity of the detection process.

A further aspect of the present invention is a diagnostic system to analyze samples with regard to the presence of contaminations comprising
AA) a diagnostic device and
BB) a computer program according to the present invention.

In a preferred system according to the present invention, said diagnostic device is a diagnostic device according to the present invention.

As mentioned before, a diagnostic device according to the present invention has at least two identical processing lanes each equipped with a sample input means and means for the analysis of said samples in order to obtain redundant diagnostic results.

Another preferred system according to the present invention further comprises the reagents necessary to analyze a plurality of samples with regard to the presence of contaminations.

## Claims

1. A safety method for a diagnostic system that analyzes a plurality of samples with regard to the presence of contaminations comprising the steps
a) taking an aliquot of a first sample,
b) dividing said aliquot into at least two aliquot parts,
c) analyzing each of said at least two aliquot parts with respect to the presence of contaminations simultaneously and
d) comparing the sample results obtained for each of said at least two aliquot parts in step c) in order to verify the presence or absence of contaminations in said sample and/or to detect failures of the diagnostic system, wherein
steps a) to d) are repeated with another sample from said plurality of samples until all samples of said plurality of samples are analyzed.

2. The safety method according to claim 1, wherein each sample comprises an internal control and said analyzing in step c) provides additional control results for each aliquot part that are optionally compared in step d) in addition to the comparison of said sample results in order to verify the presence or absence of contaminations in said sample and/or to detect failures of the diagnostic system.

3. The safety method according to claims 1 - 2, wherein said analyzing of each of said at least two aliquot parts in step c) is performed simultaneously on identical analyzers or simultaneously on one analyzer having at least two identical processing lanes.

4. The safety method according to claims 1 - 3, wherein in step a) said aliquot is a blent sample of aliquots taken from each member of a group of samples chosen from said plurality of samples, said blent sample is divided into at least two aliquot parts in step b) and the comparison of sample results in step d) is performed in order to verify the presence or absence of contaminations in said group of samples and/or to detect failures of the diagnostic system.

5. The safety method according to claims 2 - 4, wherein the presence of contaminations is verified, if
(i) each analysis in step c) provides a sample result and said sample results compared in step d) are equal or
(ii) each analysis in step c) provides a sample result, said sample results compared in step d) are different and the control results are valid or
(iii) not all analyses in step c) provide a sample result and the control results are valid.

6. The safety method according to claims 2 - 4, wherein a failure of the diagnostic system is detected, if
(i) the sample results from step c) that are compared in step d) are different or
(ii) not all control results are valid.

7. The safety method according to claims 2 - 4, wherein the absence of contaminations is verified, if no analysis in step c) provides a sample result and the control results are valid.

8. The safety method according to claims 1 - 7 further comprising a combination step e), wherein the sample results obtained for each analysis in step c) are combined to verify the presence or absence of contaminations in said sample.

9. A diagnostic device capable of analyzing samples with regard to the presence of contaminations comprising
A) at least two identical processing lanes each equipped with a sample input means and means for the analysis of said samples in order to obtain redundant diagnostic results of a sample and
B) means for the comparison of said redundant diagnostic results of the at least two identical processing lanes in order to verify the presence or absence of contaminations in said sample and/or to detect failures of the diagnostic device.

10. A computer program executable by a diagnostic device to analyze samples with regard to the presence of contaminations comprising the steps
aa) obtaining at least two redundant diagnostic results of a sample with respect to the presence of contaminations,
bb) comparing said redundant diagnostic results in order to verify the presence or absence of contaminations in said sample and/or to detect failures of the diagnostic device.

11. A diagnostic system to analyze samples with regard to the presence of contaminations comprising
AA) a diagnostic device and
BB) a computer program according to claim 10.

## Patentansprüche

1. Sicherheitsverfahren für ein diagnostisches System zur Analyse einer Vielzahl von Proben auf das Vorhandensein von Verunreinigungen, das folgende Schritte umfasst:
a) Entnahme eines Anteils einer ersten Probe,
b) Teilen des Anteils in zumindest zwei aliquote Teile,
c) gleichzeitiges Analysieren jedes der zumindest zwei aliquoten Teile auf das Vorhandensein von Verunreinigungen, und
d) Vergleichen der in Schritt c) für jeden der zumindest zwei aliquoten Teile erhaltenen Probenergebnisse zur Verifizierung des Vorhandenseins oder Fehlens von Verunreinigungen in der Probe und/oder zum Nachweis von Fehlern des diagnostischen Systems; worin
die Schritte a) bis d) mit einer anderen Probe aus der Vielzahl von Proben wiederholt werden, bis alle Proben der Vielzahl von Proben analysiert wurden.

2. Sicherheitsverfahren nach Anspruch 1, worin jede Probe eine interne Kontrolle umfasst und die Analyse in Schritt c) zusätzliche Kontrollergebnisse für jeden aliquoten Teil liefert, die in Schritt d) zusätzlich zu dem Vergleich der Probenergebnisse zur Verifizierung des Vorhandenseins oder Fehlens von Verunreinigungen in der Probe und/oder zum Nachweis von Fehlern des diagnostischen Systems fakultativ verglichen werden.

3. Sicherheitsverfahren nach Anspruch 1-2, worin die Analyse jedes der zumindest zwei aliquoten Teile in Schritt c) gleichzeitig auf identischen Analysegeräten oder gleichzeitig auf einem Analysegerät mit zumindest zwei identischen Prozessbahnen erfolgt.

4. Sicherheitsverfahren nach Anspruch 1-3, worin der aliquote Anteil in Schritt a) eine Mischprobe von aliquoten Anteilen ist, die aus jedem Mitglied einer aus der Vielzahl von Proben ausgewählten Gruppe von Proben entnommen wurden, wobei die Mischprobe in Schritt b) in zumindest zwei aliquote Teile geteilt wird und der Vergleich der Probenergebnisse in Schritt d) zur Verifizierung des Vorhandenseins oder Fehlens von Verunreinigungen in der Gruppe von Proben und/oder zum Nachweis von Fehlern des diagnostischen Systems erfolgt.

5. Sicherheitsverfahren nach Anspruch 2-4, worin das Vorhandensein von Verunreinigungen verifiziert ist, wenn
(i) jede Analyse in Schritt c) ein Probenergebnis liefert und die in Schritt d) verglichenen Probenergebnisse gleich sind, oder
(ii) jede Analyse in Schritt c) ein Probenergebnis liefert, die in Schritt d) verglichenen Probenergebnisse unterschiedlich sind und die Kontrollergebnisse gültig sind, oder
(iii) nicht alle Analysen in Schritt c) ein Probenergebnis liefern und die Kontrollergebnisse gültig sind.

6. Sicherheitsverfahren nach Anspruch 2-4, worin ein Fehler des diagnostischen Systems nachgewiesen ist, wenn
(i) die in Schritt d) verglichenen Probenergebnisse aus Schritt c) unterschiedlich sind, oder
(ii) nicht alle Kontrollergebnisse gültig sind.

7. Sicherheitsverfahren nach Anspruch 2-4, worin das Fehlen von Verunreinigungen verifiziert ist, wenn keine Analyse in Schritt c) ein Probenergebnis liefert und die Kontrollergebnisse gültig sind.

8. Sicherheitsverfahren nach Anspruch 1-7, das ferner einen Kombinationsschritt e) umfasst, worin die in Schritt c) für jede Analyse erhaltenen Probenergebnisse zur Verifizierung des Vorhandenseins oder Fehlens von Verunreinigungen in der Probe verifiziert werden.

9. Diagnostische Vorrichtung zur Analyse von Proben auf das Vorhandensein von Verunreinigungen, die Folgendes umfasst:
A) zumindest zwei identische Prozessbahnen, die jeweils mit einem Probeneingabemittel und einem Mittel zur Analyse der Proben ausgestattet sind, um redundante diagnostische Ergebnisse einer Probe zu erhalten, und
B) ein Mittel zum Vergleichen der redundanten diagnostischen Ergebnisse der zumindest zwei identischen Prozessbahnen zur Verifizierung des Vorhandenseins oder Fehlens von Verunreinigungen in der Probe und/oder zum Nachweis von Fehlern der diagnostischen Vorrichtung.

10. Computerprogramm, das von einer diagnostischen Vorrichtung zur Analyse von Proben auf das Vorhandensein von Verunreinigungen ausgeführt werden kann, das folgende Schritte umfasst:
aa) Erhalten von zumindest zwei redundanten diagnostischen Ergebnissen einer Probe im Zusammenhang mit dem Vorhandensein von Verunreinigungen,
bb) Vergleichen der redundanten diagnostischen Ergebnisse zur Verifizierung des Vorhandenseins oder Fehlens von Verunreinigungen in der Probe und/oder zum Nachweis von Fehlern des diagnostischen Systems.

11. Diagnostisches System zur Analyse von Proben auf das Vorhandensein von Verunreinigungen, das Folgendes umfasst:
AA) eine diagnostische Vorrichtung, und
BB) ein Computerprogramm nach Anspruch 10.

## Revendications

1. Procédé de sécurité pour un système de diagnostic qui analyse une pluralité d'échantillons en ce qui concerne la présence de contaminations, comprenant les étapes
a) de prélèvement d'une aliquote d'un premier échantillon,
b) de division de ladite aliquote en au moins deux parties d'aliquote,
c) d'analyse de chacune desdites au moins deux parties d'aliquote en ce qui concerne la présence de contaminations simultanément et
d) de comparaison des résultats d'échantillon obtenus pour chacune desdites au moins deux parties d'aliquote à l'étape c) afin de vérifier la présence ou l'absence de contaminations dans ledit échantillon et/ou afin de détecter des dysfonctionnements du système de diagnostic, dans lequel
les étapes a) à d) sont répétées avec un autre échantillon parmi ladite pluralité d'échantillons jusqu'à ce que tous les échantillons de ladite pluralité d'échantillons soient analysés.

2. Procédé de sécurité selon la revendication 1, dans lequel chaque échantillon comprend un contrôle interne et ladite analyse à l'étape c) offre des résultats de contrôle supplémentaires pour chaque partie d'aliquote qui sont facultativement comparés à l'étape d) en plus de la comparaison desdits résultats d'échantillon afin de vérifier la présence ou l'absence de contaminations dans ledit échantillon et/ou afin de détecter des dysfonctionnements du système de diagnostic.

3. Procédé de sécurité selon les revendications 1 - 2, dans lequel ladite analyse de chacune desdites au moins deux parties d'aliquote à l'étape c) est effectuée simultanément sur des analyseurs identiques ou simultanément sur un analyseur ayant au moins deux bandes de traitement identiques.

4. Procédé de sécurité selon les revendications 1 - 3, dans lequel à l'étape a) ladite aliquote est un échantillon mélangé d'aliquotes prises parmi chaque membre d'un groupe d'échantillons choisis parmi ladite pluralité d'échantillons, ledit échantillon mélangé est divisé en au moins deux parties d'aliquote à l'étape b) et la comparaison des résultats d'échantillon à l'étape d) est effectuée afin de vérifier la présence ou l'absence de contaminations dans ledit groupe d'échantillons et/ou afin de détecter des dysfonctionnements du système de diagnostic.

5. Procédé de sécurité selon les revendications 2 - 4, dans lequel la présence de contaminations est vérifiée, si
(i) chaque analyse à l'étape c) donne un résultat d'échantillon et lesdits résultats d'échantillons comparés à l'étape d) sont égaux, ou
(ii) chaque analyse à l'étape c) donne un résultat d'échantillon, lesdits résultats d'échantillons comparés à l'étape d) sont différents et les résultats des contrôles sont valides, ou
(iii) les analyses à l'étape c) ne donnent pas toutes un résultat d'échantillon et les résultats des contrôles sont valides.

6. Procédé de sécurité selon les revendications 2 - 4, dans lequel un dysfonctionnement du système de diagnostic est détecté, si
(i) les résultats d'échantillons à l'étape c) qui sont comparés à l'étape d) sont différents, ou
(ii) les résultats des contrôles ne sont pas tous valides.

7. Procédé de sécurité selon les revendications 2 - 4, dans lequel l'absence de contaminations est vérifiée, si aucune analyse à l'étape c) ne donne un résultat d'échantillon et les résultats des contrôles sont valides.

8. Procédé de sécurité selon les revendications 1 - 7 comprenant en outre une étape de combinaison e), dans laquelle les résultats d'échantillons obtenus pour chaque analyse à l'étape c) sont combinés pour vérifier la présence ou l'absence de contaminations dans ledit échantillon.

9. Dispositif de diagnostic apte à analyser des échantillons en ce qui concerne la présence de contaminations comprenant
A) au moins deux bandes de traitement identiques équipées chacune d'un moyen d'entrée d'échantillon et d'un moyen pour l'analyse desdits échantillons afin d'obtenir des résultats de diagnostic redondants d'un échantillon et
B) un moyen pour la comparaison desdits résultats de diagnostic redondants des au moins deux bandes de traitement identiques afin de vérifier la présence ou l'absence de contaminations dans ledit échantillon et/ou afin de détecter des dysfonctionnements du dispositif de diagnostic.

10. Programme informatique exécutable par un dispositif de diagnostic pour analyser des échantillons en ce qui concerne la présence de contaminations comprenant les étapes
aa) d'obtention d'au moins deux résultats de diagnostic redondants d'un échantillon en ce qui concerne la présence de contaminations,
bb) de comparaison desdits résultats de diagnostic redondants afin de vérifier la présence ou l'absence de contaminations dans ledit échantillon et/ou afin de détecter des dysfonctionnements du dispositif de diagnostic.

11. Système de diagnostic pour analyser des échantillons en ce qui concerne la présence de contaminations comprenant
AA) un dispositif de diagnostic et
BB) un programme informatique selon la revendication 10.
